# EUROPEAN PATENT APPLICATION

(11) **EP 4 550 510 A1**
(43) Date of publication of application: **07.05.2025**
(21) Application number: 23831893.5
(22) Date of filing: 28.06.2023
(51) Int. Cl.: H01M 10/0569, H01M 10/052, C07C 19/08, H01M 10/0525, H01M 10/0568

(54) **NON-AQUEOUS ELECTROLYTE SOLUTION FOR LITHIUM BATTERY AND LITHIUM BATTERY COMPRISING SAME**

(30) Priority: 28.06.2022 KR 20220079024
(71) Applicant: Seoul National University R&DB Foundation, Seoul 08826 (KR); University Of Fribourg, 1700 Fribourg (CH)
(72) Inventor: CHOI, Jang Wook, Seoul 06737 (KR); COSKUN, Ali, 1752 Fribourg (CH); ZHAO, Yan, 1700 Fribourg (CH); ZHOU, Tianhong, 1700 Fribourg (CH)
(74) Representative: Gille Hrabal Partnerschaftsgesellschaft mbB Patentanwälte
(86) International application number: PCT/KR2023/009009
(87) International publication number: WO 2024/005534

(57) **Abstract**

The present invention relates to an electrolyte solution for a lithium metal battery, wherein when applied to a battery including a lithium metal as a negative electrode active material, the electrolyte solution of the present invention has fewer side reactions and excellent stability, and thus, may improve overall performance of the battery.

## Description

### TECHNICAL FIELD

The present disclosure relates to a non-aqueous electrolyte solution for a lithium metal battery and a lithium metal battery including the non-aqueous electrolyte solution, wherein the non-aqueous electrolyte solution may be applied to a lithium metal battery including a lithium metal as a negative electrode active material to improve the overall performance of the battery.

### BACKGROUND ART

With the rapid development of the electronics, telecommunications and computer industries, the application of energy storage technology is expanding to camcorders, mobile phones, laptops, PCs, and even electric vehicles. Accordingly, high-performance secondary batteries, which are lightweight, long-lasting, and highly reliable, are being developed.

Among secondary batteries currently being applied, lithium secondary batteries developed in the early 1990s have been adopted as power sources for many portable devices due to high operating voltages and significantly higher energy density compared to typical batteries such as Ni-MH, Ni-Cd, and sulfuric acid-lead batteries using aqueous solution electrolytes.

As a negative electrode active material of such a lithium secondary battery, a lithium metal, a carbon-based material, silicon, or the like is used, and among these, the lithium metal has an advantage of achieving the highest energy density, and thus, is being continuously studied.

A lithium electrode using a lithium metal as an active material is typically manufactured by having flat copper or nickel foil as a current collector, and attaching lithium foil thereon. Alternatively, methods, such as using lithium foil itself as a lithium electrode without a separate current collector, or assembling a battery using only a current collector without a lithium foil, and then forming a lithium metal layer by charging and discharging the battery and using the lithium metal layer as a negative electrode (anodeless method), are known.

However, a lithium secondary battery including a lithium metal electrode has a problem in that an electrolyte solution decomposition reaction continuously occurs without forming a stable interface between an electrolyte solution and the lithium metal electrode, due to the high reactivity of a lithium metal and a surface non-uniformity phenomenon which occurs while the lithium metal is being plated on and de-laminated from the electrode during charging and discharging of the battery.

A product of the electrolyte solution decomposition reaction not only rapidly increases battery resistance by acting as a resistance layer which interferes with the deintercalation and plating of lithium, but also depletes the electrolyte solution and available lithium in the battery, causing the deterioration in lifespan of the battery.

### DISCLOSURE OF THE INVENTION

### TECHNICAL PROBLEM

The present disclosure provides a non-aqueous electrolyte solution having excellent stability for a lithium metal.

The present disclosure also provides a non-aqueous electrolyte solution having excellent stability for a battery including a negative electrode active material other than a lithium metal.

The present disclosure also provides a lithium battery including the non-aqueous electrolyte solution. The purposes of the present invention are not limited to the purposes mentioned above, and other purposes and advantages of the present invention not mentioned can be understood by the following description, and will be more clearly understood by embodiments of the present invention. In addition, it can be easily seen that the purposes and advantages of the present invention may be implemented by means and combinations thereof shown in the claims.

### TECHNICAL SOLUTION

In accordance with an exemplary embodiment of the present invention, a compound is represented by Formula 1 below.

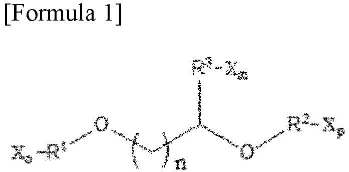

In Formula 1, R¹, R², and R³ are each independently hydrogen, or an alkyl group having 1 to 5 carbon atoms, wherein if the R¹, R², and R³ are each independently an alkyl group having 1 to 5 carbon atoms, X is F, Cl, Br, or I, n is an integer of 1 to 5, and m, o, p are each an integer of 0 to 3.

Meanwhile, in accordance with another exemplary embodiment of the present invention, a non-aqueous electrolyte solution for a lithium battery includes a solvent represented by Formula 1 below, and a lithium salt.

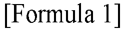

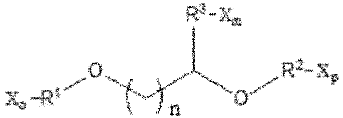

In Formula 1, R¹, R², and R³ are each independently hydrogen, or an alkyl group having 1 to 5 carbon atoms, wherein if the R1, R2, and R3 are each independently an alkyl group having 1 to 5 carbon atoms, X is F, Cl, Br, or I, n is an integer of 1 to 5, and m, o, p are each an integer of 0 to 3.

In an embodiment of the present invention, the solvent represented by Formula 1 above may be 1,1,1-trifluoro-2,3-dimethoxypropan (TFDMP) represented by Formula 1a below.

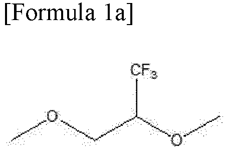

In an embodiment of the present invention, the lithium salt included in the non-aqueous electrolyte solution may be lithium bis(fluorosulfonyl)imide (LiFSI).

In an embodiment of the present invention, the concentration of the lithium salt may be approximately 0.5 M to approximately 4.0 M (mol/L).

In an embodiment of the present invention, the solvent of Formula 1 above may be included in an amount of approximately 50 vol% to approximately 100 vol% based on 100 vol% of total non-aqueous solvents.

In accordance with yet another exemplary embodiment of the present invention, a lithium secondary battery includes a positive electrode including a positive electrode active material, a negative electrode including a negative electrode active material, a separator interposed between the negative electrode and the positive electrode, and the non-aqueous electrolyte solution for a lithium battery.

In an embodiment of the present invention, the negative electrode active material may be a lithium metal.

In an embodiment of the present invention, the negative electrode active material may be a silicon-based negative electrode active material.

In an embodiment of the present invention, the positive electrode active material may be one or more selected from the group consisting of composite oxides of cobalt, manganese, nickel, aluminum, iron, or a combination thereof with lithium.

The above means for achieving the purposes do not include all the features of the present invention. Various features of the present invention and advantages and effects in accordance thereto may be understood in more detail with reference to the following specific embodiments.

### ADVANTAGEOUS EFFECTS

Effects of the non-aqueous electrolyte solution of the present invention are as follows.

The electrolyte solution of the present invention has excellent stability for a lithium metal, and thus, may suppress an electrolyte solution decomposition reaction, and therefore, when applied to a lithium metal battery, the electrolyte solution may improve the lifespan and high-rate charging performance of the battery.

In addition, the non-aqueous electrolyte solution of the present invention has excellent stability for a battery including a negative electrode active material other than a lithium metal, and thus, may improve lifespan properties and high-rate performance of the battery.

In addition to the above-described effects, specific effects of the present invention will be described together while explaining the specific details for carrying out the invention below.

### BRIEF DESCRIPTION OF THE DRAWINGS

FIG. 1 shows the chemical structural formulas and properties of solvents included in Example 1 and Comparative Examples 1 and 2 in accordance with exemplary embodiment of the present invention.
FIG. 2 shows the LSV evaluation results of electrolyte solutions of Example 1 and Comparative Example 1 and 2 in accordance with exemplary embodiment of the present invention.
FIG. 3 shows the coulombic Efficiency (CE) evaluation results of lithium metal batteries including the electrolyte solutions of Example 1 and Comparative Example 1 and 2 in accordance with exemplary embodiment of the present invention.
FIG. 4 shows FE-SEM images of aluminum surfaces identified when aluminum (Al) corrosion evaluation is performed using the electrolyte solutions of Example 1 and Comparative Examples 1 and 2 in accordance with exemplary embodiment of the present invention.
FIG. 5 shows the comparison results of full-cell performance of the lithium metal batteries including the electrolyte solutions of Example 1 and Comparative Examples 1 and 2 in accordance with exemplary embodiment of the present invention.
FIG. 6 shows the high-rate lifespan evaluation result of a lithium metal battery (NP Ratio=2.5) including the electrolyte solution of Example 1 in accordance with exemplary embodiment of the present invention.
FIG. 7 shows the high-rate lifespan evaluation result of a lithium metal battery (NP Ratio=1) including the electrolyte solution of Example 1 in accordance with exemplary embodiment of the present invention.
FIG. 8 shows the initial coulombic efficiency evaluation results of lithium batteries (including silicon as a positive electrode active material) including the electrolyte solutions of Example 2 and Comparative Example 3 in accordance with exemplary embodiment of the present invention.
FIG. 9 shows the high-rate lifespan evaluation results of lithium batteries (including silicon as a negative electrode active material) including the electrolyte solutions of Example 2 and Comparative Example 3 in accordance with exemplary embodiment of the present invention.

### MODE FOR CARRYING OUT THE INVENTION

Hereinafter, principles of preferred embodiments of the present invention will be described in detail with reference to the accompanying drawings and descriptions. However, the drawings illustrated below and the descriptions to be described later are for a preferred method among various methods for effectively explaining the features of the present invention, and the present invention is not limited only to the following drawings and descriptions.

Meanwhile, the terms "first," "second," or the like may be used for describing various elements, but these terms should be interpreted only for the purpose of distinguishing one component from the other. For example, a first element may be referred to as a second element, and a second element may also be referred to as a first element in a similar manner.

The terms of singular forms may include plural forms unless the context clearly indicates otherwise. In the present specification, it should be understood that the term "include" or "have" is intended to specify the presence of stated features, numbers, steps, operations, elements, parts, or combinations thereof described herein, but do not preclude the presence or addition of one or more other features, numbers, steps, operations, elements, parts, or combinations thereof.

Unless otherwise defined, all the terms used herein, including technical or scientific terms, have the same meanings as those commonly understood by those skilled in the art. Terms such as those defined in a dictionary commonly used should be construed as having meanings consistent with meanings in the context of the related art, and should not be construed as having ideal or overly formal meanings unless explicitly defined in the present specification.

Hereinafter, the present invention will be described in more detail.

First, in the present invention, a "lithium metal battery" means a battery which uses a lithium metal as a negative electrode.

The present invention relates to an electrolyte solution for a lithium metal battery, the electrolyte solution including a solvent represented by Formula 1 below.

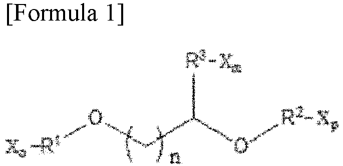

In Formula 1, R¹, R², and R³ are each independently hydrogen, or an alkyl group having 1 to 5 carbon atoms, wherein if the R1, R2, and R3 are each independently an alkyl group having 1 to 5 carbon atoms, X is F, Cl, Br, or I, n is an integer of 1 to 5, and m, o, p are each an integer of 0 to 3.

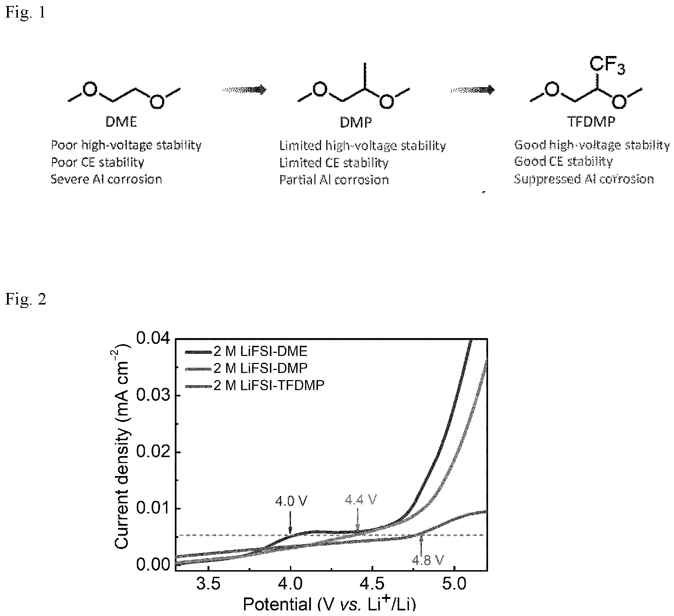

The inventors of the present invention have repeatedly conducted research on a solvent of a non-aqueous electrolyte solution suitable for use in a lithium metal battery including a lithium metal as a negative electrode active material, and as a result, have confirmed that when the solvent of Formula 1 above, in which the backbone structure of a typical 1,2-dimethoxyethane (DME) solvent is changed, is applied to a lithium metal battery, the solvent shows significantly improved stability compared to a typical electrolyte solution, and as a result, greatly improves the overall electrochemical performance including the lifespan of the lithium metal battery, and have completed the present invention (see FIG. 1).

In an embodiment of the present invention, the solvent represented by [Formula 1] above may be 1,1,1-trifluoro-2,3-dimethoxypropan (TFDMP) represented by Formula 1a below.

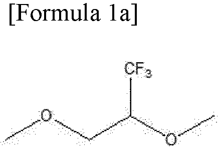

A lithium metal electrode used in a lithium metal battery is a negative electrode material highly effective in improving the energy density of a lithium-based battery due to its low reduction voltage (-3.04 V vs. SHE), high theoretical capacity (3860 mAhg⁻¹), and low density (0.544 gcm⁻³). Particularly, when the lithium metal electrode is used with a high-voltage positive electrode material, there is an advantage in that the energy density of the battery may be dramatically improved.

However, there is a problem with the lithium metal battery in that the lifespan of the battery is degraded due to the formation of lithium dendrite during a charging process and electrolyte solution consumption caused by a side reaction between an electrolyte solution and a lithium metal.

Therefore, in order to solve the above problem and achieve a high energy density at the same time, there is a demand for an electrolyte solution which is electrochemically stable to suppress the side reaction with a lithium metal, and furthermore, which has excellent oxidation stability to be used with a high-voltage positive electrode material.

A compound of Formula 1 which is used as a solvent of the non-aqueous electrolyte solution of the present invention includes a halogen-containing group (-CX₃) and an ether-containing group in one molecule. In a specific embodiment, a compound represented by Formula 1a above includes a fluorine-containing group (-CF₃) and an ether-containing group in one molecule.

In general, when a fluorine-containing group is included in a molecule, the highest occupied molecular orbital (HOMO) energy level of the molecule is lowered due to the high electronegativity of fluorine, and the lowered HOMO energy level may improve the oxidation stability of the molecule.

Particularly, the compound represented by Formula 1a, which is one of the embodiments of the present invention, has only one-group of a fluorine-containing group, thereby minimizing side effects (reduced solvation power, reduced salt solubility, etc.) which occur when the compound includes a plurality of fluorine-containing groups.

In conclusion, the compound includes a fluorine-containing group (-CF₃) in the molecule, and thus, when used as a solvent of a non-aqueous electrolyte solution, is thought to be able to improve the oxidation stability of the electrolyte solution, which has been confirmed in the LSV evaluation experiment to be described later.

If the oxidation stability of an electrolyte solution used in a lithium metal battery is secured, when a lithium metal electrode is used with a high-voltage positive electrode material, for example, a high-nickel-based positive electrode material or high-manganese or overlithiated oxide, the energy density may be remarkably improved.

In addition, a solvent used in the electrolyte solution of the present invention does not directly bond a carbon (-CX₃) in which a halogen-containing group is located with an oxygen atom from a molecular design point of view, and thus, may provide a binding site of lithium ions, which has an effect of improving the solvation power of the lithium ions. Solvents including a fluorine-containing group used in a typical lithium metal battery exhibit a phenomenon in which a carbon in which the fluorine-containing group is located is directly bonded with an oxygen atom, resulting in decreasing solvation power, but the solvent used in the electrolyte solution of the present invention has the above-described molecular design, and thus, is thought to be able to improve the solvation power of lithium ions, thereby achieving excellent electrochemical performance.

In an embodiment of the present invention, the electrolyte solution for a lithium metal battery includes lithium bis(fluorosulfonyl)imide (LiFSI) as a lithium salt.

Lithium salts which may be included in addition to LiFSI may be LiCl, LiBr, LiI, LiClO₄, LiBF₄, LiB₁₀Cl₁₀, LiPF₆, LiAsF₆, LiSbF₆, LiAlCl₄, LiSCN, LiC₄BO₈, LiCF₃CO₂, LiCH₃SO₃, LiCF₃SO₃, LiN(SO₂CF₃)₂, LiN(SO₂C₂F₅)₂, LiC₄F₉SO₃, LiC(CF₃SO₂)₃, (CF₃SO₂)₂NLi, lithium chloroborate, lower aliphatic carboxylic acid lithium, tetraphenyl boric acid lithium, lithium imide, and the like, which are typically used in an electrolyte solution.

At this time, the total concentration of LiFSI and other lithium salts in the electrolyte solution is approximately 0.5 M (mol/L) to approximately 4.0 M, preferably approximately 1.0 M to approximately 3.0 M. At a high salt concentration as described above, a side reaction between a lithium metal and an electrolyte solution may be suppressed, and effects of preventing the corrosion of a positive electrode current collector and the elution of a positive electrode active material transition metal may be secured. When the concentration of the lithium salt is too high, there may be problems of performance deterioration due to low ion conductivity and degradation in electrolyte solution wettability due to high viscosity, so that the concentration may be appropriately adjusted within the above range.

Meanwhile, the solvent of Formula 1 above may be included in an amount of approximately 50 vol% to approximately 100 vol% based on 100 vol% of total non-aqueous solvents, and the remaining volume may include a carbonate-based solvent, an ester-based solvent, and the like to be described later.

Meanwhile, the electrolyte solution of the present invention may include a cyclic fluorinated carbonate-based solvent, together with the solvent of Formula 1 above. The cyclic fluorinated carbonate-based solvent is not particularly limited as long as it is a compound in which at least two or more hydrogen are substituted with fluorine in a cyclic carbonate-based solvent typically used as a solvent of an electrolyte solution. Specifically, the cyclic fluorinated carbonate-based solvent may be one or more selected from the group consisting of fluoroethylene carbonate, difluoroethylene carbonate, and trifluoromethylethylene carbonate, and may preferably be fluoroethylene carbonate.

Meanwhile, in addition to the cyclic fluorinated carbonate-based solvent, the electrolyte solution of the present invention may include, as a non-aqueous solvent, one or more solvents (hereinafter, referred to as a chain-type solvent) selected from the group consisting of a chain-type carbonate, a chain-type ester, and a chain-type ether solvent.

The chain-type solvent may be used without limitation as long as it is a chain-type solvent typically used in an electrolyte solution for a lithium secondary battery.

Specifically, the chain-type carbonate may be one or more selected from the group consisting of dimethyl carbonate, diethyl carbonate, dipropyl carbonate, ethyl methyl carbonate, methyl propyl carbonate, and ethyl propyl carbonate.

The chain-type ester may be one or more selected from the group consisting of methyl acetate, ethyl acetate, propyl acetate, methyl propionate, ethyl propionate, and propyl propionate. The chain-type ether may be one or more selected from the group consisting of dimethyl ether, diethyl ether, dipropyl ether, methyl ethyl ether, methyl propyl ether, and ethyl propyl ether.

Preferably, one or more solvents selected from the group consisting of the chain-type carbonate, the chain-type ester, and the chain-type ether solvents may be one or more selected from the group consisting of dimethyl carbonate, diethyl carbonate, methyl propionate, ethyl propionate, dimethyl ether, and diethyl ether.

Meanwhile, if necessary, the electrolyte solution of the present invention may further include an additive for forming an SEI film in order to improve the overall performance of a battery. As the additive for forming an SEI film which may be used in the present invention, vinylene carbonate (VC), vinyl ethylene carbonate (VEC), fluoroethylene carbonate (FEC), lithium difluorophospate (LiPO2F2), lithium bis(oxalato)borate (LiBOB), lithium difluoro(oxalato)borate (LiFOB) lithium bis(fluorosulfonyl)imide (LiFSI), lithium bis(trifluoromethanesulfonyl)imide (LiTFSI), lithium difluoro bisoxalato phosphate (WCA), lithium bis(pentafluoroethylsulfonyl)amide (LiBETI), lithium(malonato oxalato)borate, LiMOB), LiPF₂C₄O₈, LiSO₃CF₃, LiPF₄(C₂O₄), LiP(C₂O₄)₃, LiC(SO₂CF₃)₃, LiBF₃(CF₃CF₂), LiPF₃(CF₃CF₂)₃, Li₂B₁₂F₁₂, 1,3-propanesultone, 1,3-propene sultone, biphenayl, cyclohexyl benzene, 4-fluorotoluene, succinic anhydride, ethylene sulfate anhydride, tris(trimethylsilyl)borate, cyclic sulfite, saturated sultone, unsaturated sultone, acyclic sulfone, and the like may be used alone, or in a mixture of two or more thereof, but the embodiment of the present invention is not limited thereto. The additive for forming an SEI film may be included in an amount of approximately 0.5 wt% to approximately 10 wt% based on the total weight of the electrolyte solution for excellent film formation.

### Lithium metal battery

According to an embodiment of the present invention, a lithium metal battery including the above-described non-aqueous electrolyte solution is provided. Specifically, the lithium metal battery includes a positive electrode, a negative electrode including a lithium metal as an active material, and a separator, and includes the electrolyte solution of the present invention as an electrolyte solution.

### (1) Positive electrode

The positive electrode includes a positive electrode active material layer formed on a positive electrode current collector. The positive electrode active material layer may include a positive electrode active material, a binder, and optionally, a conductive material.

The positive electrode current collector is not particularly limited as long as it has high conductivity without causing a chemical change in the battery, and for example, stainless steel, aluminum, nickel, titanium, fired carbon, or aluminum or stainless steel that is surface-treated with one of carbon, nickel, titanium, silver, and the like may be used. At this time, in order to increase adhesion to the positive electrode active material, the positive electrode current collector may be used in various forms, such as a film, a sheet, a foil, a net, a porous body, a foam body, and a non-woven fabric body which have microscopic irregularities formed on the surface thereof.

The positive electrode active material is a compound capable of reversible lithiation and de-lithiation of lithium, and specifically, may include a lithium composite metal oxide including one or more metals such as cobalt, manganese, nickel or aluminum, and lithium. More specifically, the lithium composite metal oxide may be a lithium-manganese-based oxide (e.g., LiMnO₂, LiMn₂O₄, etc.), a lithium-cobalt-based oxide (e.g., LiCoO₂, etc.), a lithium-nickel-based oxide (e.g., LiNiO₂, etc.), a lithium-nickel-manganese-based oxide (e.g., LiNi_{1-Y}MnyO₂ (wherein 0<Y<1), LiMn_{2-z}Ni_{z}O₄ (wherein 0 < Z < 2), etc.), a lithium-nickel-cobalt-based oxide (e.g., LiNi_{1-Y1}Co_{Y1}O₂ (wherein 0<Y1<1), etc.), a lithium-manganese-cobalt-based oxide (e.g., LiCo_{1-Y2}Mn_{Y2}O₂ (wherein 0<Y2<1), LiMn_{2-z1}Co_{z1}O₄ (wherein 0 < Z1 < 2, etc.), a lithium-nickel-manganese-cobalt-based oxide (e.g., Li(NiₚCo_{q}Mnᵣ₁)O₂ (wherein 0 < p < 1, 0 < q < 1, 0 < r1 < 1, p+q+r1=1) or Li(Niₚ₁Co_{q1}Mnᵣ₂)O₄ (wherein 0 < p1 < 2, 0 < q1 < 2, 0 < r2 < 2, p1+q1+r2=2, etc.), or a lithium-nickel-cobalt-transition metal (M) oxide (e.g., Li(Niₚ₂Co_{q2}Mnᵣ₃As₂)O₂ (wherein M is selected from the group consisting of Al, Fe, V, Cr, Ti, Ta, Mg, and Mo, and p2, q2, r3, and s2 are each an atomic fraction of stand-alone elements, wherein 0 < p2 < 1, 0 < q2 < 1, 0 < r3 < 1, 0 < s2 < 1, p2+q2+r3+s2=1, etc.) and the like, and any one thereof or a compound of two or more thereof may be included.

Among these, due to the fact that the capacity properties and stability of a battery may be increased, the lithium composite metal oxide may be LiCoO₂, LiMnO₂, LiNiO₂, a lithium nickel-manganese-cobalt oxide (e.g., Li(Ni_{1/3}Mn_{1/3}Co_{1/3})O₂, Li(Ni_{0.6}Mn_{0.2}Co_{0.2})O₂, Li(Ni_{0.5}Mn_{0.3}C_{O0.2})O₂, Li(Ni_{0.7}Mn_{0.15}Co_{0.15})O₂, Li(Ni_{0.8}Mn_{0.1}Co_{0.1})O₂, etc.), or a lithium nickel cobalt aluminum oxide (e.g., Li(Ni_{0.8}Co_{0.15}Al_{0.05})O₂, etc.).

Meanwhile, as another example, the positive electrode active material may be one or more selected from the group consisting of an elemental sulfur (S8); a disulfide compound such as Li₂Sn (n≥≥1), 2,5-dimercapto-1,3,4-thiadiazole, and 1,3,5-trithiocyanuic acid, an organic sulfur compound or a sulfur-containing compound such as a carbon-sulfur polymer ((C₂Sx)n: x=2.5 to 50, n≥≥2), and the like.

The positive electrode active material may be included in an amount of approximately 80 wt% to approximately 99 wt% based on the total weight of solids in a positive electrode slurry. At this time, when the content of the positive electrode active material is approximately 80 wt% or less, energy density is lowered, and thus, the capacity may be lowered.

The binder is used for the binding of an electrode active material with a conductive material and the binding to a current collector. Non-limiting examples of the binder may include polyvinylidene fluoride (PVDF), polyvinyl chloride (PVC), polyacrylic acid (PAA), polymethacrylic acid (PMA), polymethyl methacrylate (PMMA), polyacryl amide (PAM), polymethacryl amide, polyacrylonitrile (PAN), polymethacrylonitrile, polyimide (PI), alginic acid, alginate, chitosan, carboxymethyl cellulose (CMC), starch, hydroxypropyl cellulose, regenerated cellulose, polyvinylpyrrolidone, tetrafluoroethylene, polyethylene, polypropylene, ethylene-propylene-diene polymer (EPDM), sulfonated-EPDM, styrenebutadiene rubber (SBR), fluorine rubber, and various copolymers thereof.

The conductive material is used further improve the conductivity of an electrode active material. The conductive material is not particularly limited as long as it has conductivity without causing a chemical change in the battery. For example, graphite such as natural graphite or artificial graphite; carbon black such as Super-P, acetylene black, Ketjen black, channel black, furnace black, lamp black, and thermal black; conductive fiber such as carbon fiber and metal fiber; metal powder such as fluorocarbon powder, aluminum powder, and nickel powder; a conductive whisker such as zinc oxide and potassium titanate; a conductive metal oxide such as titanium oxide; or a polyphenylene derivative, and the like may be used.

### (2) Negative electrode

The negative electrode of the present invention is a lithium metal negative electrode having a lithium metal as a negative electrode active material. The lithium metal negative electrode used when assembling the lithium metal battery may be composed only of a current collector, in the form in which a current collector is coated with a lithium metal, or composed only of a lithium metal.

The negative electrode current collector is not particularly limited as long as it has a conductivity without causing a chemical change in a battery, and for example, copper, stainless steel, aluminum, nickel, titanium, fired carbon, copper or stainless steel that is surface-treated with one of carbon, nickel, titanium, silver, and the like, an aluminum-cadmium alloy, and the like may be used. In addition, the negative electrode current collector may be used in various forms such as a film, a sheet, a foil, a net, a porous body, a foam, and a non-woven body which have microscopic irregularities formed or not formed on the surface thereof. As an example, copper foil may be used as the negative electrode current collector, but the embodiment of the present invention is not limited thereto.

The thickness of the current collector is not particularly limited, but a thickness of approximately 5 to approximately 100 µm is preferable, and a thickness of approximately 5 to approximately 50 µm is more preferable. When the thickness of the current collector is less than approximately 5 µm, it may be difficult to handle during a process, and when greater than approximately 100 µm, the thickness and weight of the battery are unnecessarily increased, which may affect the battery performance by decreasing energy density, and the like, so that the above range is preferable.

When assembling a battery, when an electrode composed only of a current collector is used as a negative electrode, lithium ions transferred from a positive electrode by initial charging and discharging after the battery assembly are irreversibly plated on the negative electrode current collector to form a lithium metal layer, and then the lithium metal layer may act as a negative electrode active material layer. At this time, when the electrode composed only of a current collector is used as the negative electrode, the current collector may include a metal and metal oxide particles to induce uniform plating.

Alternatively, a negative electrode including a lithium metal, which is an active material, may be used from the time of battery assembly, at which time, a method for coating the lithium metal on a negative electrode current collector is not particularly limited. As an example, a method of laminating a thin film of the lithium metal on the current collector and then roll-pressing, a method of electrolytic or electroless plating of the lithium metal on the current collector, and the like may be used. At this time, the thickness of the lithium metal layer of the negative electrode is not particularly limited, but may be approximately 10 µm or greater, or approximately 20 µm to approximately 50 µm, or approximately 40 µm or less.

In the case of a lithium metal negative electrode composed only of a lithium metal, the thickness thereof is not particularly limited, but may be approximately 10 µm or greater, or approximately 20 µm to approximately 50 µm, or approximately 40 µm or less.

Meanwhile, according to another embodiment of the present invention, the non-aqueous electrolyte solution described above may be applied to a negative electrode other than a lithium metal.

Specifically, the solvent of the present invention may be applied to an electrode in which a silicon-based active material, for example, Si or SiO x, is used as a negative electrode active material. In this case, the negative electrode is manufactured by coating a mixture of a negative electrode active material, a conductive material, and a binder on a negative electrode current collector and then drying and pressing the mixture.

### (3) Separator

The separator is to separate the negative electrode and the positive electrode and to provide a movement path for lithium ions, and any separator may be used as long as it is a separator typically used in a lithium battery. That is, a separator having excellent electrolyte solution impregnation of an electrolyte solution as well as low resistance to ion movement in the electrolyte solution may be used. For example, a separator selected from glass fiber, polyester, Teflon, polyethylene, polypropylene, polytetrafluoroethylene (PTFE), or a combination thereof and in the form of a nonwoven fabric or a woven fabric may be used.

For example, a separator including a polyolefin-based polymer such as polyethylene and polypropylene, or a separator including a coating layer containing a ceramic component or a polymer material to secure heat resistance or mechanical strength may be used, and such a separator may be used in a single-layered or a multi-layered structure. In an embodiment, as the separator, a separator prepared by coating a ceramic coating material containing ceramic particles and an ionic binder polymer on both sides of a polyolefin-based polymer substrate may be used.

According to an embodiment of the present invention, there is provided a lithium metal battery including the positive electrode, the negative electrode, and the separator described above, and the electrolyte solution of the present invention. At this time, the shape of the lithium metal battery is not particularly limited, and may have various shapes such as a cylindrical shape, a pouch shape, and a coin shape.

Hereinafter, preferred embodiments of the present invention will be described in detail to facilitate understanding of the present invention. However, the following embodiments are merely illustrative of the present invention, and thus, it will be apparent to those skilled in the art that various modifications and variations can be made without departing from the scope and spirit of the present invention as disclosed in the accompanying claims. Also, it is obvious that such variations and modifications fall within the scope of the appended claims. Hereinafter, Examples and Comparative Examples of the present invention will be described. However, the following Examples are only illustrative of the present invention, and the present invention is not limited to the following Examples.

### [Examples]

### Manufacturing Example 1 (Manufacturing of TFDMP electrode)

600 mL of dry THF and 1 mol (24 g) of NaH were introduced to a 1 L roundbottom flask, and stirred for 20 min under a 0°C argon atmosphere. Thereafter, 0.4 mol (54 g) of 1,1,1-trifluoro-2,3-propanediol was slowly added thereto by using a syringe pump. After the 1,1,1-trifluoro-2,3-propanediol was introduced, stirring was further performed for 1 hour at 0 °C to prepare a mixture. Then, 1 mol (62.4 mL) of iodomethane was added dropwise to the mixture under a 0 °C condition. The mixture was stirred at room temperature for 2 hours and refluxed overnight while being slowly heated. Thereafter, a liquid portion of the mixture was fractionally distilled using a 60 cm fractionation column, and the distillation process was performed three times to obtain a final product (TFDMP).

### (1) Preparation of non-aqueous electrolyte solution

Electrolyte solutions of Examples and Comparative Examples were prepared in the compositions of Table 1 below. In the table below, DME means 1,2-dimethoxyethane, DMP means 1,2-dimethoxypropane, EC means ethylene carbonate, DEC means di-ethyl carbonate, and FEC means fluoroethylene carbonate. The concentration (M) of a lithium salt is the total number of moles (mol) of the lithium salt per 1 L of the total solvent included in the electrolyte solution. All the electrolyte solutions were prepared in a glove box filled with an inert gas.

**[Table 1]**

| | Lithium salt | | Solvent | | | | Additive |
|---|---|---|---|---|---|---|---|
| | LiFSI | LiPF6 | DME | DMP | TFDMP | EC/DEC | FEC |
| Comparative Example 1 | 2.0M | | 0 | | | | |
| Comparative Example 2 | 2.0M | | | 0 | | | |
| Comparative Example 3 | | 1.0M | | | | 0 | 0 |
| Example 1 | 2.0M | | | | 0 | | |
| Example 2 | 3.0M | | | | 0 | | |

### (2) Manufacturing of positive electrode

NCM 811 (LiNi_{0.8}Co_{0.1}Mn_{0.1}O₂) as a positive electrode active material, Super-P as a conductive material, polyvinylidene fluoride (PVdF) as a binder, and N-methylpyrrolidone as a solvent were used to prepare a positive electrode active material slurry having a weight ratio of active material:conductive material:binder of 8:1:1. Next, the positive electrode active material slurry was coated on one surface of an aluminum foil, followed by roll-pressing and drying, to manufacture a positive electrode having a loading of approximately 5.0 mg/cm².

### (3) Manufacturing of negative electrode

### Lithium metal electrode

Lithium metal foil (manufactured by China Energy Lithium Corporation) having a thickness of approximately 20 µm was used as a negative electrode.

### Silicon electrode

Micro Si was used as negative electrode active material to manufacture a negative electrode. Specifically, Micro Si as a negative electrode active material, carboxymethylcellulose (CMC) as a binder, Super-P as a conductive material, and distilled water were used to prepare a negative electrode active material slurry having a weight ratio of 92:4:4 of active material:conductive material:binder. Then, the negative electrode active material slurry was coated on one side of a copper foil, roll-pressed and dried to manufacture a negative electrode having a loading of 5.0 mg/cm².

### (4) Manufacturing of full-cell

A separator and the lithium metal foil of (3) were stacked on the positive electrode of (2) to manufacture a Swagelok-type cell or a 2032-type coin-cell in which the negative electrode/separator/positive electrode were sequentially stacked in the order in a glove box. As the separator, Cellgard 2400 (manufactured by Celgard Corporation) composed of polyethylene was used, and an N/P ratio was set to 2.5, and approximately 40 µl of the electrolyte solution of each of Example 1 and Comparative Examples 1 and 2 was injected to manufacture a full-cell.

A separator and the Micro Si electrode of (3) were stacked on the negative electrode of (2) to manufacture a Swagelok-type cell or a 2032-type coin-cell in which the negative electrode/separator/positive electrode were sequentially stacked in the order in a glove box. As the separator, Cellgard 2400 (manufactured by Celgard Corporation) composed of polyethylene was used, and an N/P ratio was set to 2.5, and approximately 40 µl of the electrolyte solution of each of Example 2 and Comparative Example 3 was injected to manufacture a full-cell.

### Evaluation Example 1. Evaluation of oxidation stability

An oxidation stability evaluation experiment was performed on the electrolyte solution of each of Example 1 and Comparative Examples 1 and 2. Oxidation stability was evaluated by performing the linear sweep voltammetry (LSV) analysis method. Specifically, a Li/Al half-cell was manufactured, and then the electrolyte solution of each of Example 1 and Comparative Examples 1 and 2 was injected thereto, followed by performing the LSV analysis method at a scan rate of approximately 0.5 mVs⁻¹ to a range of approximately 5.0 V (vs.Li/Li⁺) in an open circuit voltage (OCV). The evaluation results are shown in FIG. 2.

Referring to FIG. 1, the electrolyte solution of Example 1 (TFDMP) did not initiate a large oxidation current up to about 4.8 V (vs.Li/Li⁺), whereas the electrolyte solution of Comparative Example 1 (DME) and the electrolyte solution of Comparative Example 2 (DMP) rapidly initiated an oxidation current around of about 4.0 V (vs.Li/Li⁺) and around 4.4V (vs.Li/Li⁺), respectively. From the result, it can be confirmed that an electrolyte solution including the solvent of the present invention has excellent oxidation stability.

### Evaluation Example 2. Evaluation of coulombic efficiency

In order to confirm the reversibility when the electrolyte of each of Example 1 and Comparative Examples 2 and 3 was used in a lithium metal battery, the coulombic efficiency was evaluated.

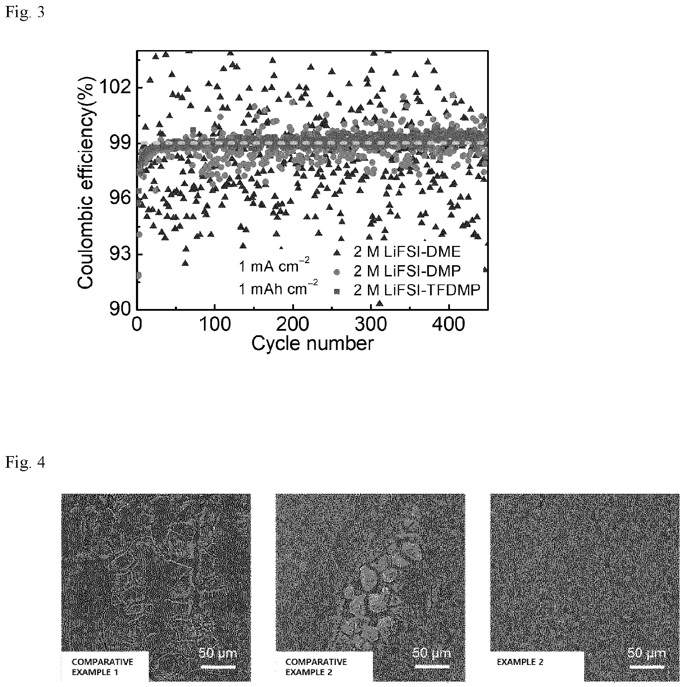

Specifically, a Li/Cu half-cell was manufactured, and then the electrolyte solution of each of Example 1 and Comparative Examples 2 and 3 was introduced thereto to evaluate the coulombic efficiency. In the Li/Cu half-cell, depending on a current applied, lithium ions can be plated and re-striped on the Cu, and the ratio between an amount of lithium ions plated and an amount thereof stripped was calculated as the coulombic efficiency, and the results are shown in FIG. 3.

Referring to FIG. 3, it can be confirmed that the half-cells including the electrolyte solutions of Comparative Examples 1 and 2 show extreme fluctuations in the coulombic efficiency in the entire cycle region, which is determined to be due to unstable SEI generation and irreversible lithium ion plating. On the other hand, it was confirmed that the half-cell including the electrolyte solution of Example 1 showed relatively stable coulombic efficiency in the entire cycle region.

### Evaluation Example 3. Evaluation of Al corrosion

In order to evaluate the oxidation stability of the electrolyte solutions of Example 1 and Comparative Examples 1 and 2, Al corrosion evaluation was performed. Aluminum foil is used as a current collector of a positive electrode, and if the surface of aluminum is corroded due to a reaction between aluminum and an electrolyte solution under an oxidation condition, the performance of the positive electrode may be adversely affected by an increase in surface resistance, etc.

Specifically, a Li/Al half-cell was manufactured, the electrolyte solution of each of Example 1 and Comparative Examples 1 and 2 were introduced thereto, and then 5.0 V (vs.Li/Li⁺) was applied for 24 hours (constant voltage experiment). Thereafter, the half-cell was decomposed, and the surface of aluminum was observed using field emission scanning electron microscopy (FE-SEM; Tescan Mira3 LM FE) to confirm the corrosion of aluminum (FIG. 4).

FIG. 4 shows the surface of a half-cell using the electrolyte solution of each of Example 1 and Comparative Examples 1 and 2 in accordance with exemplary embodiment of the present invention. The half-cells using the electrolyte solutions of Comparative Examples 1 and 2 showed very severe corrosion (e.g., cracks, flakes) on the aluminum surface, whereas the aluminum of the half-cell using the electrolyte solution of Example 1 showed a very smooth surface without cracks.

### Evaluation Example 4. Evaluation of full-cell performance

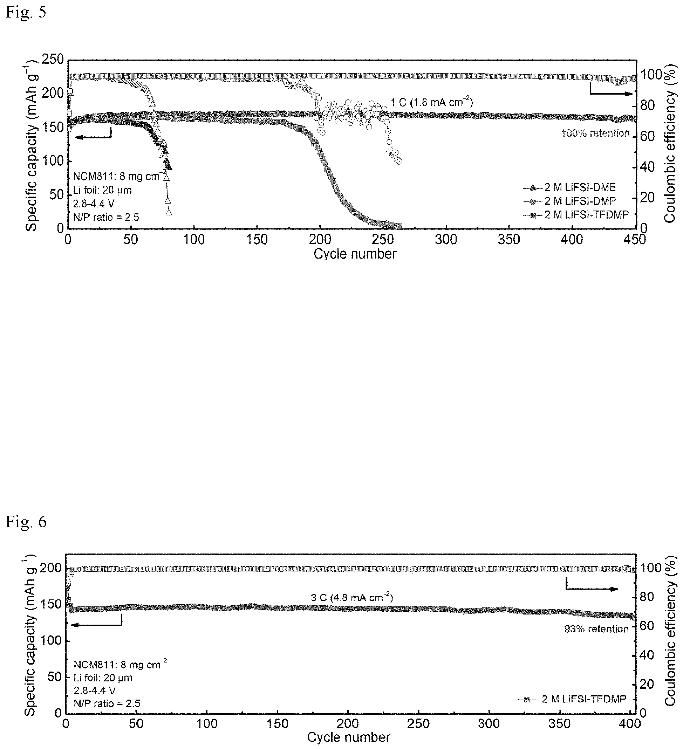

A full-cell including the electrolyte solution of each of Example 1 and Comparative Examples 1 and 2 was manufactured, and a current of 1.0 C-rate (1.6 mA cm⁻²) was applied thereto to perform charge/discharge, and the lifespan performance of the full-cell was evaluated, and the results are shown in FIG. 5. The cell containing the electrolyte solution of Example 1 showed excellent stability by showing 100% retention up to 450 cycles, whereas the cell containing the electrolyte solution of Comparative Example 1 was confirmed to have deterioration in lifespan performance within 60 cycles, and the cell containing the electrolyte solution of Comparative Example 2 was confirmed to have deterioration in lifespan performance within 175 cycles.

Meanwhile, in order to observe the high-rate lifespan performance of a cell containing the electrolyte solution of the present invention, the lifespan performance of a full-cell (NP ratio = 2.5) containing the electrolyte solution of Example 1 was evaluated while applying a current of 3.0 C-rate (4.8 mA cm⁻²) thereto, and the results are shown in FIG. 6. The cell containing the electrolyte solution of the present invention showed a retention of 93% at 550 cycles even under a high-current charge/discharge condition such as 3.0 C-rate (4.8 mA cm⁻²), and was confirmed to exhibit excellent performance.

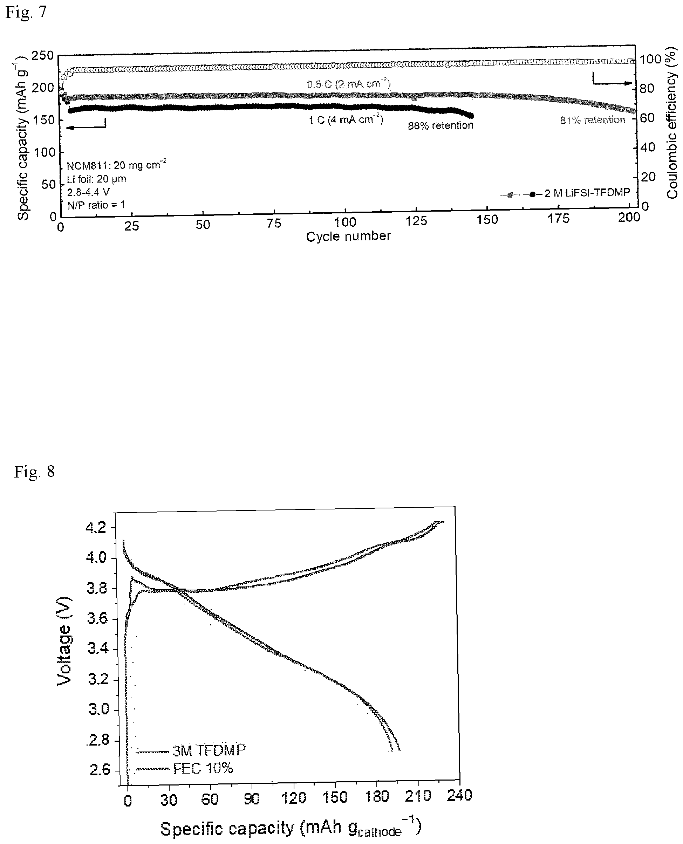

In addition, while applying a current of 0.5 C-rate (2 mA cm⁻²) and 1.0 C-rate (4.0 mA cm⁻²) to a full-cell (NP Ratio=1) containing the electrolyte solution of Example 1, the lifespan performance of the full-cell was evaluated, and the results are shown in FIG. 7. The cell containing the electrolyte solution of the present invention shows a retention of 81% of at 200 cycles under a current application condition of 0.5 C-rate (2 mA cm⁻²), and showed a retention of 88% at 145 cycles under a current application condition of 1.0 C-rate (4.0 mA cm⁻²).

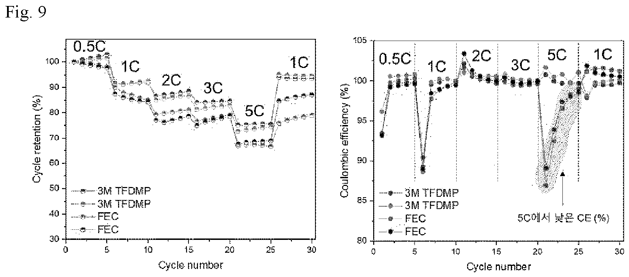

Full-cells containing the electrolyte solutions of Example 2 and Comparative Example 3 were manufactured, and the initial coulombic efficiency and high-rate lifetime performance of each of the cells were observed, and the results are shown in FIG. 8 and FIG. 9.

The cell containing the electrolyte solution of Example 2 showed initial coulombic efficiency of about 85.2%, and the cell containing Comparative Example 3, which has a typical electrolyte solution composition, showed initial coulombic efficiency of about 83.5% (see FIG. 8).

Meanwhile, the cell containing the electrolyte solution of Example 2 showed a retention of about 75% even at a high rate of 5.0 C-rate, and also showed stable coulombic efficiency throughout the cycles, whereas the cell containing the electrolyte solution of Comparative Example 3, which has a typical electrolyte solution composition, showed retention of about 67%, and at the same time, was confirmed to have low coulombic efficiency observed at a high rate (FIG. 9).

From these results, it was confirmed that the electrolyte solution containing the solvent of the present invention is capable of improving the electrochemical performance of a lithium metal battery or a battery containing silicon as a negative electrode active material.

It is to be understood that the above-described embodiments are exemplary in all respects and are not intended to be limiting, and that the scope of the present invention is represented by the appended claims rather than in the detailed description, and all changes or modifications derived from the meaning and range of the patent registration claims and their equivalent concepts should be interpreted as being included in the scope of the present invention.

## Claims

1. A compound represented by Formula 1 below: 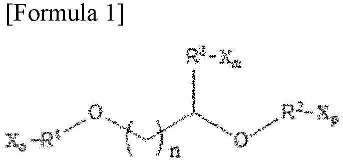 wherein in Formula 1,
R¹, R², and R³ are each independently hydrogen, or an alkyl group having 1 to 5 carbon atoms, wherein if the R1, R2, and R3 are each independently an alkyl group having 1 to 5 carbon atoms, X is F, Cl, Br, or I, n is an integer of 1 to 5, and
m, o, p are each an integer of 0 to 3.

2. A non-aqueous electrolyte solution for a lithium battery, comprising: a solvent represented by Formula 1 below; and a lithium salt: 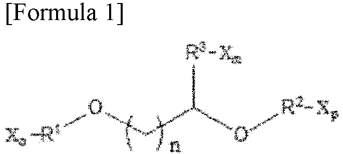 wherein in Formula 1,
R¹, R², and R³ are each independently hydrogen, or an alkyl group having 1 to 5 carbon atoms, wherein if the R1, R2, and R3 are each independently an alkyl group having 1 to 5 carbon atoms, X is F, Cl, Br, or I, n is an integer of 1 to 5, and
m, o, p are each an integer of 0 to 3.

3. The non-aqueous electrolyte solution of claim 2, wherein the solvent represented by Formula 1 above is a compound represented by Formula 1a below:

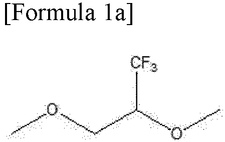

4. The non-aqueous electrolyte solution of claim 2, wherein the lithium salt is lithium bis(fluorosulfonyl)imide.

5. The non-aqueous electrolyte solution of claim 4, wherein the lithium salt has a concentration of approximately 0.5 M to approximately 4.0 M (mol/L).

6. The non-aqueous electrolyte solution of claim 2, wherein the solvent of Formula 1 above is included in an amount of approximately 50 vol% to approximately 100 vol% based on 100 vol% of total non-aqueous solvents.

7. A lithium battery comprising:
a positive electrode including a positive electrode active material;
a negative electrode including a negative electrode active material;
a separator interposed between the negative electrode and the positive electrode; and
the non-aqueous electrolyte solution for a lithium metal battery of claim 2.

8. The lithium battery of claim 7, wherein the negative electrode active material is a lithium metal.

9. The lithium battery of claim 7, wherein the negative electrode active material comprises a silicon-based active material.

10. The lithium battery of claim 7, wherein the positive electrode active material is one or more selected from the group consisting of composite oxides of cobalt, manganese,
nickel, aluminum, iron, or a combination thereof with lithium.
